# EUROPEAN PATENT SPECIFICATION

(11) **EP 1 835 951 B1**
(45) Date of publication and mention of the grant of the patent: **02.02.2011**
(21) Application number: 05854593.0
(22) Date of filing: 16.12.2005
(51) Int. Cl.: A61M 5/142

(54) **HCV inhibitor for use in a method of treating hepatitis C**
HCV Inhibitor zur Verwendung in einem Verfahren zur Behandlung von Hepatitis C
Inhibiteur du VHC à utiliser dans un procédé de traitement de l`hépatite C

(30) Priority: 20.12.2004 US 637477 P
(43) Date of publication of application: 26.09.2007
(73) Proprietor: CODMAN & SHURTLEFF INC., Raynham, MA 02767-0350 (US); Tibotec Pharmaceuticals, Co Cork (IE)
(72) Inventor: ZHAO, Ruilin, San Diego, California 92128 (US); BAERT, Lieven, E., C., B-8200 Brugge (BE); SIMMEN, Kenneth, A., B-3080 Tervuren (BE)
(74) Representative: Tunstall, Christopher Stephen
(86) International application number: PCT/US2005/045913
(87) International publication number: WO 2006/068996

(56) References cited:
- WO-A-00/59929
- WO-A-03/087092
- US-A- 5 318 519
- US-A- 5 752 930
- US-A1- 2003 045 861
- US-B1- 6 638 263

## Description

### CROSS-REFERENCE TO RELATED APPLICATIONS

This application is a non-provisional application which claims priority to previously filed provisional application U S. Serial No. 60/637,477, filed December 20, 2004, entitled A Method and System for Treating Hepatitis C

### BACKGROUND OF THE INVENTION

### 1. Field of the Invention

The present invention relates to at least one HCV inhibitor for use in a method of treating Hepatitis C. More particularly, the present invention relates to at least one HCV inhibitor for use in a method of treating Hepatitis C with an implantable infusion pump to deliver antiviral therapeutics directly to the liver.

### 2. Discussion of Related Art

The Hepatitis C virus (HCV) is one of the most important causes of chronic liver disease. Hepatitis C accounts for about 15% of acute viral hepatitis, 60 to 70% of chronic hepatitis, and up to 50% of cirrhosis, end stage liver disease, and liver cancer. Almost 4 million Americans have antibody to HCV (anti-HCV), indicating ongoing or previous infection with the virus.

Following initial acute infection, a majority of infected individuals develop chronic hepatitis because HCV replicates preferentially in hepatocytes but is not directly cytopathic. In particular, the lack of a vigorous T-lymphocyte response and the high propensity of the virus to mutate appear to promote a high rate of chronic infection. Chronic hepatitis can progress to liver fibrosis leading to cirrhosis, end-stage liver disease, and HCC (hepatocellular carcinoma), making it the leading cause of liver transplantations.

There are 6 major HCV genotypes and more than 50 subtypes, which are differently distributed geographically. HCV type 1 is the predominant genotype in Europe and the US. The extensive genetic heterogeneity of HCV has important diagnostic and clinical implications, perhaps explaining difficulties in vaccine development and the lack of response to therapy.

Transmission of HCV can occur through contact with contaminated blood or blood products, for example following blood transfusion or intravenous drug use. The introduction of diagnostic tests used in blood screening has led to a downward trend in post-transfusion HCV incidence. However, given the slow progression to the end-stage liver disease, the existing infections will continue to present a serious medical and economic burden for decades.

Current HCV therapies are based on (pegylated) interferon-alpha (IFN-α) in combination with ribavirin. This combination therapy yields a sustained virologic response in more than 40% of patients infected by genotype 1 viruses and about 80% of those infected by genotypes 2 and 3. Beside the limited efficacy on HCV type 1, this combination therapy has significant side effects and is poorly tolerated in many patients. Major side effects include influenza-like symptoms, hematologic abnormalities, and neuropsychiatric symptoms. Hence there is a need for more effective, convenient and better tolerated treatments.

Recently, peptidomimetic HCV protease inhibitors have gained attention as clinical candidates, namely BILN-2061 disclosed in WO 00/59929 and VX-950 disclosed in WO 03/87092. A number of similar HCV protease inhibitors have also been disclosed in the academic and patent literature. It has already become apparent that the sustained administration of these agents selects HCV mutants in in *vitro* HCV replicon models, which are resistant to the respective drug, so called drug escape mutants. Accordingly, additional drugs with different resistance patterns may be required to provide failing patients with treatment options, and combination therapy with multiple drugs is likely to be the norm in the future, even for first line treatment.

Experience with HIV drugs, and HIV protease inhibitors in particular, has learned that sub-optimal pharmacokinetics and complex dosing regimes often result in inadvertent compliance failures. The 24 hour trough concentration (minimum plasma concentration) of the respective drugs in an HIV regime frequently falls below the IC₉₀ or ED₉₀ threshold for large parts of the day thereby causing the emergence of drug escape mutants. The same applies to HCV therapy. Ideally, the drug plasma concentrations of the anti-viral agent should not only be kept above such trough levels and by preference should be more or less stable without much variation. Providing anti-HCV therapy that complies with these requirements therefore is a highly desirable goal to achieve.

There is a need for new treatments of HCV, which may overcome one or more of the disadvantages of current HCV therapy such as side effects, limited efficacy, emergence of resistance, and compliance failures.

### SUMMARY OF THE INVENTION

In accordance with a currently preferred exemplary embodiment, the present invention uses a least one HCV inhibitor in a method of treating HCV by locally delivering medication directly to the liver by way of an implantable drug pump having an outlet catheter delivering medication directly to the desired location in the liver or to any body vessel outside the liver, which drains body fluid into the liver.

Thus in one aspect, the present invention concerns at least one HVC inhibitor for use in a method of treating a patient infected with HCV, said method comprising the steps of : filling an implantable drug infusion pump with at least one HCV inhibitor, locally delivering an HCV inhibitory effective amount of one or more HCV inhibitors directly to the liver by way of an implantable drug infusion pump having an outlet catheter delivering the one or more HCV inhibitors directly to the desired location in the liver or to any body vessel outside the liver, which drains body fluid into the liver.

### BRIEF DESCRIPTION OF THE DRAWINGS

The above and still Further objects, features and advantages of the present invention will become apparent upon consideration of the following detailed description of a specific aspect thereof, especially when taken is conjunction with the accompanying drawings wherein like reference numerals in the various figures are utilized to designate like components, and wherein:

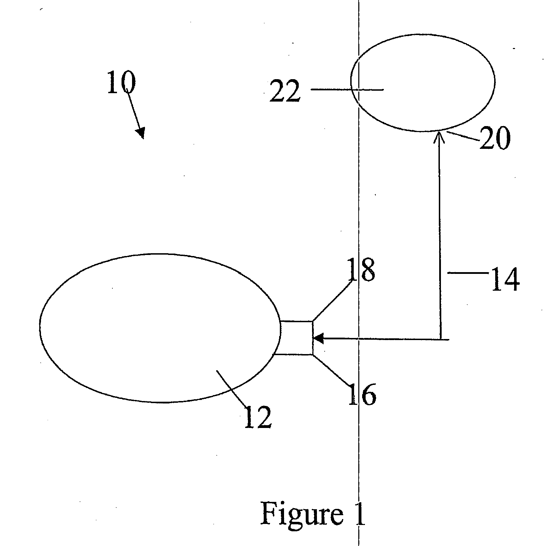

Figure 1 is a schematic view of an implantable drug pump delivering medication via a catheter to the liver.

### DETAILED DESCRIPTION OF THE INVENTION

Referring now to Figure 1, a method and system 10 for treating Hepatitis C is illustrated. The method and system includes using an implantable pump 12 and an outlet catheter 14. A proximal end 16 of outlet catheter 14 is fluidly connected to an outlet port 18 of pump 12. A distal end 20 of outlet catheter 14 is sized to deliver medication from pump 12 to a liver 22 of living organist, preferably a human being.

As used herein delivering directly to the liver means that the medication is delivered at a site of the liver or at any body vessel outside the liver that drains body fluid into the liver. The medication can be, for example, delivered to the liver or upstream from the liver vascular bed or at any other infusion location as desired by the physician. In one embodiment, the medication is delivered to a vein of the liver, preferably the portal vein (vena porta).

Any HCV inhibitory compound can be used in the method of treating hepatitis C in accordance with the present invention. HCV inhibitory agents comprise, for instance, interferon-α (IFN-α), albumin-fused interferons, pegylated interferon-α and/or ribavirin, as well as therapeutics based on antibodies targeted against HCV epitopes, small interfering RNA (Si RNA) as well as vector-encoded short hairpin RNA (shRNA), ribozymes, DNAzymes, antisense RNA, small molecule antagonists of for instance NS3 protease, NS3 helicase and NS5B polymerase or any other HCV non structural or structural protein. Other therapeutic agents that can be used include immunomodulatory compounds targeting the human TOLL-like receptors (e.g. the TLR-7 and TLR-9 receptors), which also lead to a decrease in HCV viral load. These are currently nucleic acid based and a number of such agents have entered clinical development, and typically are delivered by injection (Actilon, Coley Pharmaceuticals; Isatoribine, Anadys Pharmaceuticals). Some other HCV inhibitory compounds, such as, for example, small interfering RNA (Si RNA), have only been administered intravenously. The present inventors have discovered that some HCV inhibitory compounds including small interfering RNA (Si RNA) can be used in the method of treating hepatitis C in accordance with the present invention. Si RNAs have recently been shown to be effective in clearing HCV viral RNA from mammalian cells harboring HCV genomes (Randall, Grakoui and Rice Proceedings of the National Academy of Sciences, 100; 2003, pp235-24). Whether such Si RNAs will deliver therapeutic benefit in patients will be largely dependent on their specific delivery to the target liver organ, and the possibilities of side effects on other organs. The challenges in the delivery of nucleic acid based therapeutics are well documented (see McHutchison and Patel, Hepatology, 36, 2002, Suppl.1 S245-S252, and Lee et al, Hepatology, 32, 2000, pp640-646) and include the rapid breakdown of such agents by nucleases present in serum. The pharmacokinetics and distribution of the Heptazyme HCV specific ribozyme RPI.13919 (RPI), designed to cleave the HCV IRES sequence, have shown that it targets to the liver, but delivery to other organs is thought to contribute to extra-hepatic toxicity in primates. While Si RNAs have yet to be used in the clinic in HCV patients, there are many reports of their successful use in cellular models. Delivery of such nucleic acid agents direct to the liver via implantable drug pump pumps as in accordance with the present invention may offer advantages of reduced extra-hepatic toxicity and enhanced delivery to the site of antiviral action.

Similarly, ISIS-14803 is an antisense oligonucleotide that entered clinical development for treatment of HCV infected patients, but has since been halted because of limited efficacy and adverse effects. The present invention may allow using these agents in HCV therapy.

Of interest are protease inhibitors such as BILN-2061 disclosed in WO 00/59929 and Vertex' VX-950 disclosed in WO 03/87092. Other protease inhibitors are GS 9132, also known as ACH-806. (Gilead/Achillion) and SCH-503034 (Schering). Further agents that can be used are those disclosed in WO-98/17679. WO-00/056331 (Vertex); WO 98/22496 (Roche); WO 99/07734, (Boehringer Ingelheim ), WO 2005/073216 WO2005073195 (Medivir) and structurally similar agents. Other agents that can be used are the imino sugar derivative, UT231B (United Therapeutics) that appears to block the activity of the p7 HCV protein and the NS5B polymerase inhibitor NM283 (Valopicitabine; Idenix/Novartis), an oral prodrug of 2'-C-methyl-cytidine. Other agents that can be used are the non-nuelcoside polymerase inhibitors (NNIs) such as JTK-109 and JTK-003 (Japan Tobacco), and R803 (Rigel), HCV-371, HCV-086 and HCV-796 (ViroPharma/Wyeth) Further agents that can be used are nucleoside analogues with the potential to complement or replace ribavirin such as viramidine (Valeant Pharmaceucticals), a less toxic pro-drug of ribavirin, and levovirin, an L-isomer of ribavirin, merimepodib (Vertex), an IMPDH inhibitor.

Also combinations of HCV inhibitory agents may be used for purposes of combination therapy. The term "combination therapy" relates to the administration of more than one anti-HCV compound, which may be administered as a combined preparation for simultaneous administration, or for separate or sequential administration. Current standard of care of HCV comprises combination therapy with pegylated interferon-α and ribavirin. Other combinations comprise any of the above mentioned nucleosides with pegylated interferon.

By delivering medication directly to the liver the effects of Hepatitis C are more effectively reduced. Additionally, the side effects, as compared to conventional methods of treatment are also reduced. Conventional methods comprise the systemic administration of HCV inhibitory agents thereby causing the spread of these agents at various parts of the body where their presence is not necessary and may cause side effects. The present invention allows a more targeted administration of the HCV agents in that these are directly delivered to the liver, which is the site where the Hepatitis C virus exerts its negative effects.

Moreover, this mode of administration is less prone to pharmacokinetical problems, which in particular in case of oral administration can reduce the effectiveness of the active ingredient. A more targeted administration as in the system and method of the present invention allow lower doses of the active ingredient to be administered for the drug to be effective, thereby reducing the risk of undesired side effects. In instances where the drug is less effective or the diagnosis of the disease requires the present invention allows the administration of increased doses of the drug with limited risk of side effects.

Additionally, the present invention allows for a better control of the drug plasma concentrations of the anti-viral agent in that these levels not only can be kept in the safe area above the minimum plasma levels ('trough' levels) below which the virus is able to mutate. The present invention furthermore allows the keeping of the plasma levels stable without much variation, thereby avoiding undesired side effects.

The HCV inhibitors for use in the method of the invention preferably are formulated into liquid formulations that are suited for parenteral administration. In these formulations, the carrier will usually comprise sterile water, while other ingredient, for example, to aid solubility, may be included. The carrier may comprise saline solution, glucose solution or a mixture of saline and glucose solution. The carrier may further comprise appropriate liquid carriers, suspending agents and the like. This type of formulation is desired because in preferred embodiments, the pump is refilled by injection through the skin into an appropriate inlet into the pump's drug reservoir.

Various types of drug administration pumps can be used with the present invention. In one embodiment use is made of the Archimedes^{™} implantable pump and another type is the Codman 3000^{™} pump, both of which are commercially available from Codman & Shurtleff of Raynham, Mass. Other implantable pumps, whether constant flow pumps or programmable variable flow pumps, that may be available in the future may be used as.well. Additionally, peristaltic type implantable pumps may also be used with the present invention.

Having described the presently preferred exemplary embodiment at least one HCV inhibitor for use in a method treating Hepatitis C it is believed that other modifications, variations and changes will be suggested to those skilled in the art in view of the teachings set forth herein. Substitutions of elements from one described embodiment to another are also fully intended and contemplated. It is also to be understood that the drawing is not necessarily drawn to scale, but that it is merely conceptual in nature. It is, therefore, to be understood that all such modifications, variations, and changes are believed to fall within the scope of the present invention as defined by the appended claims.

## Claims

1. At least one HCV inhibitor for use in a method of treating hepatitis C, the method comprising the steps of:
filling an implantable drug infusion pump with the at least one HCV inhibitor;
locally delivering an HCV inhibitory effective amount of the at least one HCV inhibitor directly to the liver by way of an implantable drug infusion pump having an outlet catheter delivering the at least one HCV inhibitor directly to the desired location in the liver or to any body vessel outside the liver that drains body fluid into the liver.

2. The HCV inhibitor of claim 1, wherein the catheter delivers the at least one HCV inhibitor directly to the portal vein.

3. The HCV inhibitor of claim 1, wherein the at least one HCV inhibitor includes small interfering RNA (Si RNA).

4. The HCV inhibitor of claim 1, wherein the infusion pump is a bellows reservoir pump.

5. The HCV inhibitor of claim 1, wherein the infusion pump is a peristaltic pump.

## Patentansprüche

1. Mindestens ein HCV-Inhibitor zur Verwendung bei einem Verfahren zum Behandeln von Hepatitis C, wobei das Verfahren die folgenden Schritte umfasst:
Füllen einer implantierbaren Arzneimittelinfusionspumpe mit dem mindestens einen HCV-Inhibitor;
lokales Abgeben einer HCV wirksam hemmenden Menge des mindestens einen HCV-Inhibitors direkt an die Leber durch eine implantierbare Arzneimittelinfusionspumpe mit einem Auslasskatheter, der den mindestens einen HCV-Inhibitor direkt an den gewünschten Ort in der Leber oder an jedes Körpergefäß außerhalb der Leber, das Körperflüssigkeit in die Leber abführt, abgibt.

2. HCV-Inhibitor nach Anspruch 1, wobei der Katheter den mindestens einen HCV-Inhibitor direkt an die Pfortader abgibt.

3. HCV-Inhibitor nach Anspruch 1, wobei der mindestens eine HCV-Inhibitor *small interfering* RNA (siRNA) einschließt.

4. HCV-Inhibitor nach Anspruch 1, wobei die Infusionspumpe eine Balgreservoirpumpe ist.

5. HCV-Inhibitor nach Anspruch 1, wobei die Infusionspumpe eine peristaltische Pumpe ist.

## Revendications

1. Au moins un inhibiteur du VHC à utiliser dans une méthode de traitement de l'hépatite C, la méthode comprenant les étapes consistant à :
✔ remplir une pompe à perfusion de médicament implantable avec l'au moins un inhibiteur du VHC ;
✔ délivrer localement une quantité efficace inhibitrice du VHC de l'au moins un inhibiteur du VHC directement vers le foie au moyen d'une pompe à perfusion de médicament implantable ayant un cathéter de sortie délivrant l'au moins un inhibiteur du VHC directement à l'endroit souhaité dans le foie ou à n'importe quel vaisseau corporel à l'extérieur du foie qui draine le fluide corporel dans le foie.

2. Inhibiteur du VHC selon la revendication 1, dans lequel le cathéter délivre l'au moins un inhibiteur du VHC directement à la veine porte.

3. Inhibiteur du VHC selon la revendication 1, dans lequel l'au moins un inhibiteur du VHC comprend un petit ARN interférent (ARNsi).

4. Inhibiteur du VHC selon la revendication 1, dans lequel la pompe à perfusion est une pompe à réservoir à soufflet.

5. Inhibiteur du VHC selon la revendication 1, dans lequel la pompe à perfusion est une pompe péristaltique.
